# FASCICULE DE BREVET EUROPEEN

(11) **EP 1 564 202 B1**
(45) Date de publication et mention de la délivrance du brevet: **10.09.2008**
(21) Numéro de dépôt: 05290308.5
(22) Date de dépôt: 11.02.2005
(51) Int. Cl.: C07C 233/18

(54) **Nouveau procédé de synthèse et nouvelle forme cristalline de l'agomelatine ainsi que les compositions pharmaceutiques qui la contiennent**
Neues Verfahren zur Herstellung und eine neue Kristallform des Agomelatins sowie pharmazeutische Zusammensetzungen, die diese enthalten
Novel process for synthesizing and a novel crystal form of agomelatin as well as pharmaceutical preparations containing these

(30) Priorité: 13.02.2004 FR 0401439
(43) Date de publication de la demande: 17.08.2005
(73) Titulaire: Les Laboratoires Servier, 92415 Courbevoie Cédex (FR)
(72) Inventeur: Souvie, Jean-Claude, 76600 Le Havre (FR); Gonzalez Blanco, Isaac, 45002 Toledo (ES); Thominot, Gilles, 76640 Normanville (FR); Chapuis, Geneviève, 45000 Orleans (FR); Horvath, Stéphane, 45380 La Chapelle-Saint.Mesmin (FR); Damien, Gérard, 45130 Meung-Sur-Loire (FR)

(56) Documents cités:
- EP-A- 0 447 285
- US-A- 3 931 188
- US-A- 3 992 403
- DEPREUX P ET AL: "SYNTHESIS AND STRUCTURE-ACTIVITY RELATIONSHIPS OF NOVEL NAPHTHALENIC AND BIOISOSTERIC AMIDIC DERIVATIVES AS MELATONIN RECEPTOR LIGANDS" JOURNAL OF MEDICINAL CHEMISTRY, AMERICAN CHEMICAL SOCIETY. WASHINGTON, US, vol. 37, no. 20, 30 septembre 1994 (1994-09-30), pages 3231-3239, XP002016146 ISSN: 0022-2623
- TINANT, BERNARD ET AL: "N-[2-(7-Methoxy-1-naphthyl)ethyl]acetamid e, a potent melatonin analog" ACTA CRYSTALLOGRAPHICA, SECTION C: CRYSTAL STRUCTURE COMMUNICATIONS , C50(6), 907-10 CODEN: ACSCEE; ISSN: 0108-2701, 1994, XP009047983

## Description

La présente invention concerne un procédé de synthèse industriel de l'agomélatine ou *N*-[2-(7-méthoxy-1-naphtyl)éthyl]acétamide de formule (I) :

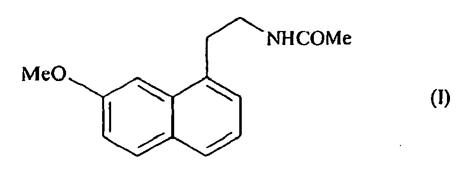

La présente invention concerne également la forme cristalline II de l'agomélatine, son procédé de préparation ainsi que les compositions pharmaceutiques qui la contiennent.

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confère une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Il était également important de pouvoir accéder à l'agomélatine sous une forme cristalline bien définie, parfaitement reproductible et présentant des caractéristiques intéressantes de filtration et de facilité de formulation.

Le brevet EP 0 447 285 décrit l'accès en huit étapes à l'agomélatine à partir de la 7-méthoxy-1-tétralone avec un rendement moyen inférieur à 30%.
Ce procédé implique l'action du bromoacétate d'éthyle, suivie d'une aromatisation et saponification pour conduire à l'acide correspondant, qui est ensuite transformé en acétamide puis déshydraté pour conduire au (7-méthoxy-1-naphtyl)acétonitrile, suivie d'une réduction puis de la condensation du chlorure d'acétyle.

En particulier, l'accès au (7-méthoxy-1-naphtyl)acétonitrile implique six étapes réctionnelles et, transposé à l'échelle industrielle, il a rapidement été mis en évidence des difficultés de mise en oeuvre de ce procédé dues principalement à des problèmes de reproductibilité de la première étape constituée par l'action du bromoacétate d'éthyle sur la 7-méthoxy-1-tétralone selon la réaction de Réformatsky conduisant au (7-méthoxy-3,4-dihydro-1(2*H*)-naphtalenylidène)éthanoate d'éthyle.
De plus, l'étape suivante d'aromatisation du (7-méthoxy-3,4-dihydro-1(2*H*)-naphtalenylidène)éthanoate d'éthyle était souvent partielle et conduisait, après saponification à un mélange de produits difficilement purifiable.

La littérature décrit l'accès en trois étapes au (7-méthoxy-1-naphtyl)acétonitrile à partir de la 7-méthoxy-1-tétralone par action de LiCH₂CN suivie d'une déshydrogénation au DDQ (2,3-dichloro-5,6-dicyano-1,4-benzoquinone) et enfin d'une déshydratation en milieu acide (Synthetic Communication, 2001, 31(4), 621-629). Toutefois le rendement global est moyen (76%) et surtout le DDQ utilisé dans la réaction de déshydrogénation ainsi que le reflux de benzène nécessaire à la troisième étape ne répondent pas aux contraintes industrielles de coût et d'environnement.

La demanderesse a présentement mis au point un nouveau procédé de synthèse industriel qui conduit, de façon reproductible et sans nécessiter de purification laborieuse, à l'agomélatine avec une pureté qui est compatible avec son utilisation comme principe actif pharmaceutique.

Une alternative aux difficultés rencontrées avec le procédé décrit dans le brevet EP 0 447 285 a été obtenue en condensant directement un dérivé cyano sur la 7-méthoxy-1-tétralone.

Il fallait de plus que le composé de condensation obtenu puisse être facilement soumis à une aromatisation afin de conduire au (7-méthoxy-1-naphtyl)acétonitrile sans nécessiter de conditions drastiques et permette l'utilisation de réactifs compatibles avec les exigences industrielles de coût et d'environnement.

Il est apparu que le (7-méthoxy-3,4-dihydro-1-naphtalényl)acétonitrile constituerait un intermédiaire de synthèse idéal répondant aux exigences requises de synthèse directe à partir de la 7-méthoxy-1-tétralone et serait un excellent substrat pour l'étape d'aromatisation.

Des condensations directes de tétralones avec l'acétonitrile ou des dérivés d'acétonitrile sont décrites dans la littérature. En particulier, le brevet US 3,992,403 décrit la condensation de cyanométhylphosphonate sur la 6-fluoro-1-tétralone, et le brevet US 3,931,188 décrit la condensation de l'acétonitrile sur la tétralone conduisant à l'intermédiaire cyané qui est directement engagé dans la réaction suivante.
Appliqué à la 7-méthoxy-1-tétralone, la condensation de l'acétonitrile conduit à un mélange d'isomères « exo » majoritaire et « endo » minoritaire selon la figure 1 : 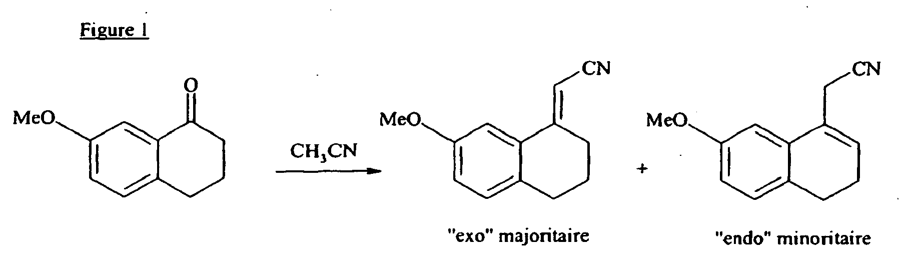 ce mélange nécessitant des conditions ultérieures d'aromatisation drastiques non compatibles avec les exigences industrielles pour poursuivre la synthèse de l'agomélatine.

La demanderesse a présentement mis au point un nouveau procédé de synthèse industriel permettant d'obtenir le (7-méthoxy-1-naphtyl)acétonitrile de façon reproductible et sans nécessiter de purification laborieuse, en deux étapes seulement à partir de la 7-méthoxy-
tétralone en utilisant comme intermédiaire de synthèse le (7-méthoxy-3,4-dihydro-1-naphtalényl)acétonitrile exempt de l'impureté « exo » de formule (II) : 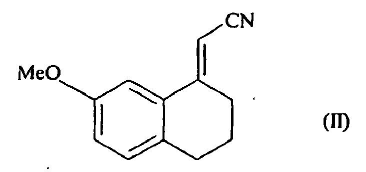 qui ne peut être soumis à la réaction d'aromatisation ultérieure dans des conditions opératoires compatibles avec les exigences industrielles afin de poursuivre la synthèse de l'agomélatine.

Plus spécifiquement, la présente invention concerne un procédé de synthèse industriel du composé de formule (I) : 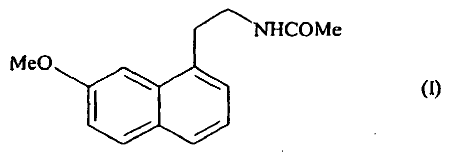 caractérisé en ce que l'on met en réaction la 7-méthoxy-1-tétralone de formule (III) : 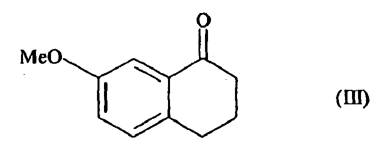 avec l'acide cyanoacétique de formule (IV) : 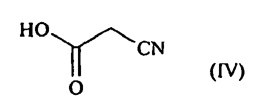 dans des conditions d'élimination de l'eau formée, en présence d'une quantité catalytique du composé de formule (V) : 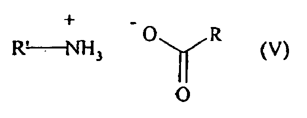 dans laquelle R et R', identiques ou différents, représentent chacun un groupement alkyle (C₃-C₁₀) linéaire ou ramifié, un groupement aryle non substitué ou substitué, ou un groupement arylalkyle (C₁-C₆) linéaire ou ramifié non substitué ou substitué,
pour conduire après filtration et lavage par une solution basique au (7-méthoxy-3,4-dihydro-1-naphtalényl)acétonitrile de formule (VI) : 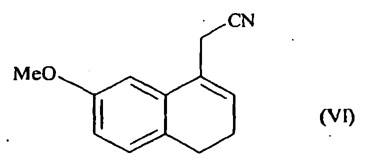 composé de formule (VI) qui est mis en réaction avec un catalyseur d'hydrogénation en présence d'un dérivé allylique pour conduire au composé de formule (VII) : 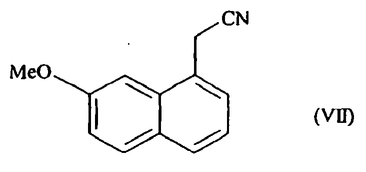 qui est ensuite soumis à une réduction par l'hydrogène en présence de Nickel de Raney dans un milieu éthanol ammoniacal, puis salifié avec de l'acide chlorhydrique pour conduire au composé de formule (VIII) : 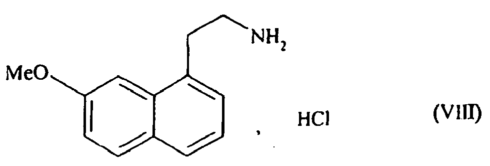 qui est successivement soumis à l'action d'acétate de sodium puis d'anhydride acétique pour conduire au composé de formule (I) que l'on isole sous la forme d'un solide,
étant entendu que :
- par aryle on entend un groupement phényle, naphtyle ou biphényle,
- le terme « substitué » affecté aux expressions « aryle » et « arylalkyle » signifie que la partie aromatique de ces groupements peut être substituée par 1 à 3 groupements, identiques ou différents, choisis parmi alkyle (C₁-C₆) linéaire ou ramifié, hydroxy, et alkoxy (C₁-C₆) linéaire ou ramifié,
- par « dérivé allylique » on entend toute molécule contenant 3 à 10 atomes de carbone et pouvant contenir en plus 1 à 5 atomes d'oxygène, et contenant au moins un motif-CH₂-CH=CH₂.

Plus particulièrement, dans la réaction de transformation du composé de formule (III) en composé de formule (VI), l'eau formée est éliminée par distillation. On utilise préférentiellement un solvant de réaction ayant une température d'ébullition supérieure ou égale à celle de l'eau et encore plus préférentiellement formant un azéotrope avec l'eau comme par exemple le xylène, le toluène, l'anisole, l'éthylbenzène, le tétrachloroéthylène, le cyclohexène, ou le mésitylène.
De façon préférée, la réaction de transformation du composé de formule (III) en composé de formule (VI) est réalisée au reflux du toluène ou du xylène et plus préférentiellement au reflux du toluène.

Avantageusement, dans la réaction de transformation du composé de formule (III) en composé de formule (VI), l'un des groupements R ou R' du catalyseur utilisé représente un groupement alkyle (C₃-C₁₀) linéaire ou ramifié, et l'autre représente un groupement aryle ou arylalkyle. Plus particulièrement, un catalyseur préféré est celui de formule (Vₐ) : 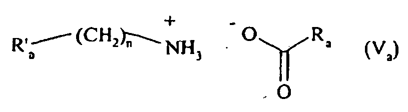 dans laquelle R'ₐ représente un groupement phényle non substitué ou substitué par un ou plusieurs groupements alkyle (C₁-C₆) linéaire ou ramifié, n vaut 0 ou 1, et Rₐ représente un groupement alkyle (C₃-C₁₀) linéaire.

Très avantageusement, R'ₐ représente un groupement phényle non substitué ou substitué et plus particulièrement un groupement phényle non substitué.

Le groupement Rₐ préféré est le groupement hexyle.

La valeur préférée de n est 1.

Le catalyseur préféré utilisé dans la réaction de transformation du composé de formule (III) en composé de formule (VI) selon le procédé de l'invention est l'heptanoate de benzylammonium de formule (IX) :

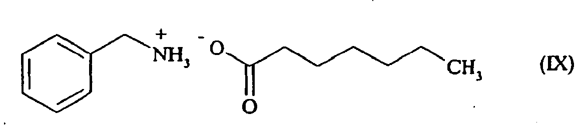

Avantageusement, le composé de formule (VI) est obtenu après filtration et lavage par une solution basique organique ou minérale comme NaOH, KOH, Ca(OH)₂, Sr(OH)₂, ou NH₄OH, et plus préférentiellement par une solution d'hydroxyde de sodium.

De façon préférentielle, la réaction de transformation du composé de formule (VI) en composé de formule (VII) est réalisée au reflux du toluène ou du xylène et plus préférentiellement au reflux du toluène.

Le catalyseur utilisé préférentiellement dans la réaction de transformation du composé de formule (VI) en composé de formule (VII) est un catalyseur sous forme d'oxyde ou supporté comme par exemple le palladium, le platine, le nickel, A1₂O₃ et plus particulièrement le palladium. Avantageusement, on utilisera le palladium sur charbon, plus particulièrement le palladium sur charbon de 1 à 20% et encore plus particulièrement à 5% ou 10%. Préférentiellement, on utilisera du palladium sur charbon dans des quantités catalytiques, plus particulièrement dans des quantités allant de 1 à 10% en poids de catalyseur par rapport au poids de substrat et plus préférentiellement 5%.
L'accepteur d'hydrogène utilisé préférentiellement dans la réaction de transformation du composé de formule (VI) en composé de formule (VII) est un dérivé allylique et plus particulièrement un acrylate d'allyle ou un allylglycidyléther. L'acrylate d'allyle préféré du procédé selon l'invention est le méthacrylate d'allyle.

Avantageusement, la réaction de transformation du composé de formule (VII) en composé de formule (VIII) selon le procédé de l'invention est réalisé entre 20 et 40°C et plus préférentiellement entre 30 et 40°C, et encore plus avantageusement à 40°C.

De façon avantageuse, la réaction de transformation du composé de formule (VIII) en composé de formule (I) est réalisée en milieu alcoolique et plus particulièrement en milieu éthanolique.

Ce procédé est particulièrement intéressant pour les raisons suivantes :
- il permet d'obtenir à l'échelle industrielle le composé « endo » de formule (VI) de façon exclusive. Ce résultat est tout à fait surprenant lorsqu'on considère la littérature concernant ce type de réaction qui fait le plus souvent état de l'obtention de mélanges « exo »/ « endo » (Tetrahedron, 1966, 22, 3021-3026). Ce résultat provient de l'utilisation d'un composé de formule (V) comme catalyseur de la réaction en lieu et place des acétates d'ammonium couramment utilisés dans ces réactions (Bull. Soc. Chim. Fr., 1949, 884-890).
- le taux de transformation du composé de formule (III) en composé de formule (VI) obtenu est très élevé, supérieur à 97% contrairement à ce qui a pu être observé avec l'utilisation d'acide acétique pour lequel ce taux ne dépasse pas 75%.
- l'utilisation d'un catalyseur d'hydrogénation en présence d'un dérivé allylique et plus particulièrement du méthacrylate d'allyle pour la transformation du composé de formule (VI) en composé de formule (VII) est tout à fait compatible avec les exigences industrielles de coût et d'environnement, contrairement aux quinones couramment utilisées.
- il permet de plus d'obtenir à l'échelle industrielle le composé de formule (VII) de façon exclusive, en particulier exempt du produit de réduction correspondant de formule (X) : 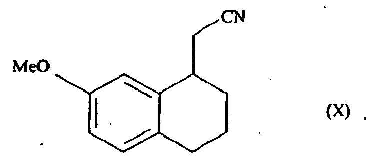
- enfin les taux de transformations du composé de formule (VI) en composé de formule (VII) observés sont élevés, supérieurs à 90%.
- l'hydrogénation du composé de formule (VII) en présence de Nickel de Raney en milieu éthanol ammoniacal est décrite (J. Med. Chem., 1994, 37(20), 3231-3239) mais nécessite des conditions difficilement transposables à l'échelle industrielle : la réaction est réalisée à 60°C et pendant 15 heures, et le rendement final est inférieur à 90%. Par ailleurs, le principal inconvénient de cette réaction est la formation concomitante du dérivé « bis » de formule (XI) : 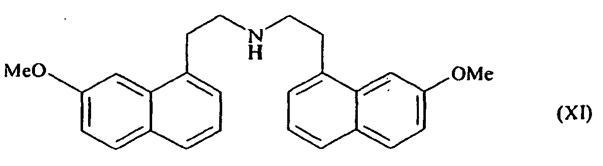 et la difficulté est de maîtriser le taux de formation de cette impureté. Le procédé mis au point par la demanderesse permets d'obtenir le composé de formule (VIII) avec un taux d'impureté bis inférieur à 4% avec des conditions expérimentales compatibles avec les exigences industrielles puisque la réaction est réalisée entre 30 et 40°C pour conduire à un rendement supérieur à 90% et une pureté chimique supérieure à 99,5%.
- l'étape d'amidification réalisée en milieu alcoolique et plus particulièrement éthanolique permet d'isoler le composé de formule (I) très facilement avec un rendement quantitatif. Ce résultat est totalement surprenant car ce type de réaction est peu compatible avec ce solvant pour lequel on s'attend à une consommation compétitive de l'anhydride acétique.

Le composé de formule (VI) obtenu selon le procédé de l'invention est nouveau et est utile en tant qu'intermédiaire de synthèse de l'agomélatine dans laquelle il est soumis à une réaction d'aromatisation suivie d'une réaction de réduction puis de couplage avec l'anhydride acétique.

L'invention s'étend également à la forme cristalline II de l'agomélatine obtenue selon le procédé précédemment décrit. Il est en effet important de pouvoir obtenir une forme cristalline bien définie et parfaitement reproductible.

L'art antérieur EP0447285 et Yous et al. (Journal of Medicinal Chemistry, 1992, 35 (8), 1484-1486) permet d'accéder à l'agomélatine sous une forme cristalline particulière qui a été décrite dans Tinant et al. (Acta Cryst., 1994, C50, 907-910).

La demanderesse a présentement mis au point un procédé d'obtention de l'agomélatine sous une forme cristalline bien définie, parfaitement reproductible et présentant de ce fait des caractéristiques intéressantes de filtration et de facilité de formulation.

Plus spécifiquement, la présente invention concerne la forme cristalline II de l'agomélatine, caractérisée par les paramètres suivants, obtenus à partir du diagramme de poudre effectué sur le diffractomètre haute résolution D8 de Bruker AXS avec un domaine angulaire 3°-90° en 2θ, un pas de 0,01° et 30 s par pas :
- maille cristalline monoclinique,
- paramètres de maille : a = 20,0903 Å, b = 9,3194 Å, c = 15,4796 Å, β = 108,667°
- groupe d'espace : P2₁/n
- nombre de molécules dans la maille : 8
- volume de la maille : Vₘₐᵢₗₗₑ = 2746,742 Å³
- densité : d = 1,13 g/cm³.

L'obtention de cette forme cristalline a pour avantage de permettre une filtration particulièrement rapide et efficace, ainsi que la préparation de formulations pharmaceutiques ayant une composition constante et reproductible, ce qui est particulièrement avantageux lorsque ces formulations sont destinées à l'administration orale.

La forme ainsi obtenue est suffisamment stable pour autoriser son stockage prolongé sans conditions particulières de température, de lumière, d'humidité ou de taux d'oxygène.

L'étude pharmacologique de la forme ainsi obtenue a montré une importante activité sur le système nerveux central ainsi que sur la microcirculation qui permet d'établir son utilité dans le traitement du stress, des troubles du sommeil, de l'anxiété, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, la forme II de l'agomélatine peut être utilisée dans les dysfonctionnements sexuels, qu'elle possède des propriétés d'inhibiteurs de l'ovulation, d'immunomodulateurs et qu'elle est susceptible d'être utilisée dans le traitement des cancers.

La forme cristalline II de l'agomélatine sera utilisée de préférence dans les traitements de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'invention s'étend aussi aux compositions pharmaceutiques renfermant comme principe actif la forme cristalline II de l'agomélatine avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,1 mg à 1 g par jour en une ou plusieurs prises.

### Exemple 1 : N-[2-(7-Méthoxy-1-naphtyl)éthyl]acétamide

### Stade A : (7-Méthoxy-3,4-dihydro-1-naphtalényl)acétonitrile

Dans un réacteur de 670 l sont introduits 85,0 kg de 7-méthoxy-1-tétralone, 60,3 kg d'acide cyanoacétique et 15,6 kg d'acide heptanoïque dans du toluène en présence de 12,7 kg de benzylamine. Le milieu est porté à reflux. Lorsque tout le substrat de départ a disparu, la solution est refroidie et filtrée. Le précipité obtenu est lavé par du toluène puis le filtrat obtenu est lavé par une solution de soude 2N, puis par de l'eau jusqu'à neutralité. Après évaporation du solvant, le solide obtenu est recristallisé dans un mélange éthanol/eau (80/20) pour conduire au produit du titre avec un rendement de 90% et une pureté chimique supérieure à 99%.

### Point de fusion : 48-50°C

### Stade B : (7-Méthoxy-1-naphtyl)acétonitrile

Dans un réacteur de 670 l sont introduits 12,6 kg de palladium sur charbon à 5% dans du toluène et portés à reflux, puis 96,1 kg de (7-méthoxy-3,4-dihydro-1-naphtalényl) acétonitrile en solution dans du toluène sont ajoutés ainsi que 63,7 kg de méthacrylate d'allyle. La réaction se poursuit à reflux et est suivie par chromatographie en phase vapeur. Lorsque tout le substrat de départ a disparu, le milieu réactionnel est refroidi à l'ambiante puis filtré. Après évaporation du toluène, le résidu solide obtenu est recristallisé dans un mélange éthanol/eau (80/20) pour conduire au produit du titre avec un rendement de 91% et une pureté chimique supérieure à 99%.

### Point de fusion : 83°C

### Stade C: 2-(7-Méthoxy-1-naphtyl)ethanamine, chlorhydrate

Dans un réacteur de 1100 l sont introduits 80,0 kg du composé obtenu au stade B et 24,0 kg de Nickel de Raney dans de l'éthanol et 170 l d'ammoniaque. Le milieu est agité et mis sous une pression d'hydrogène de 30 bars, puis porté à 40°C. Lorsque tout le substrat de départ a disparu, le solvant est évaporé et le résidu obtenu est remis en solution dans de l'acétate d'éthyle et 41,5 l d'une solution d'acide chlorhydrique 11N sont ajoutés. Après filtration, le précipité obtenu est lavé par de l'acétate d'éthyle puis séché en étuve pour conduire au produit du titre avec un rendement de 95,3 % et une pureté chimique supérieure à 99,5%.

### Point de fusion : 243°C

### Stade D : N-[2-(7-Méthoxy-1-naphtyl)étliyl]acétamide

Dans un réacteur de 1600 l sont introduits 173 kg du composé obtenu au stade C et 66 kg d'acétate de sodium dans de l'éthanol. Le milieu est agité puis 79 kg d'anhydride acétique sont additionnés, le milieu réactionnel est porté à reflux et 600 l d'eau sont ajoutés. La réaction est laissée revenir à l'ambiante et le précipité obtenu est filtré, lavé par un mélange éthanol/eau 35/65 pour conduire au produit du titre avec un rendement de 92,5% et une pureté chimique supérieure à 99%.

### Point de fusion : 108°C

### Exemple 2 : N-[2-(7-Méthoxy-1-naphtyl)éthyl]acétamide

### Stade A : (7-Méthoxy-3,4-dihydro-1-naphtalényl)acétonitrile

Dans un réacteur de 670 l sont introduits 85,0 kg de 7-méthoxy-1-tétralone, 60,3 kg d'acide cyanoacétique et 15,6 kg d'acide heptanoïque dans du toluène en présence de 11,0 kg d'aniline. Le milieu est porté à reflux. Lorsque tout le substrat de départ a disparu, la solution est refroidie et filtrée. Le précipité obtenu est lavé par du toluène puis le filtrat obtenu est lavé par une solution de soude 2N, puis par de l'eau jusqu'à neutralité. Après évaporation du solvant, le solide obtenu est recristallisé dans un mélange éthanol/eau (80/20) pour conduire au produit du titre avec un rendement de 87% et une pureté chimique supérieure à 99%.

### Point de fusion : 48-50°C

### Stade B : (7-Méthoxy-1-naphtyl)acétonitrile

On procède comme dans le stade B de l'Exemple 1.

### Point de fusion : 83°C

### Stade C : 2-(7-Méthoxy-1-naphtyl)ethanamine, chlorhydrate

On procède comme dans le stade C de l'Exemple 1.

### Point de fusion : 243°C

### Stade D : N-[2-(7-Méthoxy-1-naphtyl)éthyl]acétamide

On procède comme dans le stade D de l'Exemple 1.

### Point de fusion : 108°C

### Exemple 3 : Forme cristalline II du N-[2-(7-Méthoxy-1-naphtyl)éthyl]acétamide

L'enregistrement des données a été effectué sur le diffractomètre haute résolution D8 de Bruker AXS avec les paramètres suivants : un domaine angulaire 3°-90° en 2θ, un pas de 0,01° et 30 s par pas. La poudre de *N*-[2-(7-Méthoxy-1-naphtyl)éthyl]acétamide obtenue dans l'Exemple 1 a été déposée sur un support pour un montage en transmission. La source de rayons X est un tube au cuivre (λCuK_{α1} = 1,54056 Å). Le montage comporte un monochromateur avant (cristal de Ge(111)) et un détecteur solide résolu en énergie (MXP-D1, Moxtec-SEPH).
Le composé est bien cristallisé : la largeur des raies à mi-hauteur est de l'ordre de 0,07° en 2θ.

Les paramètres suivants ont ainsi été déterminés :
- maille cristalline monoclinique,
- paramètres de maille : a = 20,0903 Å, b = 9,3194 Å, c = 15,4796 Å, β = 108,667°
- groupe d'espace : P2₁/n
- nombre de molécules dans la maille : 8
- volume de la maille : Vₘₐᵢₗₗₑ = 2746,742 Å³
- densité : d = 1,13 g/_{cm}³.

### Example 4 : Composition pharmaceutique

| Formule de préparation pour 1000 comprimés doses à 25 mg : | |
|---|---|
| Composé de l'exemple 3 | 25 g |
| Lactose monohydrate | 62 g |
| Stéarate de Magnésium | 1,3 g |
| Amidon de maïs | 26 g |
| Maltodextrines | 9 g |
| Silice colloïdale anhydre | 0,3 g |
| Amidon de maïs prégélatinisé type A | 4 g |
| Acide stéarique | 2,6 g |

### Example 5 : Composition pharmaceutique

| Formule de préparation pour 1000 comprimés doses à 25 mg : | |
|---|---|
| Composé de l'exemple 3 | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

## Revendications

1. Procédé de synthèse industriel du composé de formule (I) 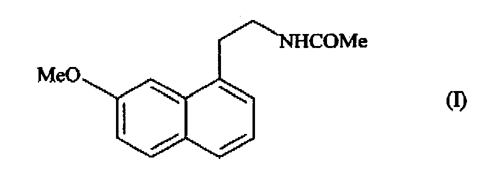 **caractérisé en ce que** l'on met en réaction la 7-méthoxy-1-tétralone de formule (III) : 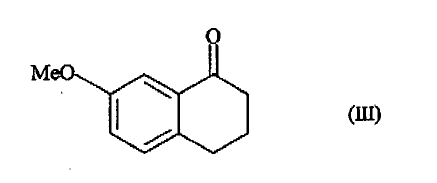 avec l'acide cyanoacétique de formule (IV) : 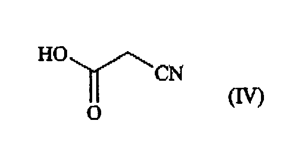 dans des conditions d'élimination de l'eau formée, en présence d'une quantité catalytique du composé de formule (V) : 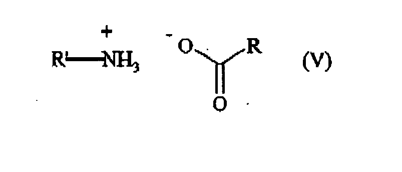 dans laquelle R et R', identiques ou différents, représentent chacun un groupement alkyle (C₃-C₁₀) linéaire ou ramifié, un groupement aryle non substitué ou substitué, ou un groupement arylalkyle (C₁-C₆) linéaire ou ramifié non substitué ou substitué, pour conduire après filtration et lavage par une solution basique au (7-méthoxy-3,4-dihydro-1-naphtalényl)acétonitrile de formule (VI) : 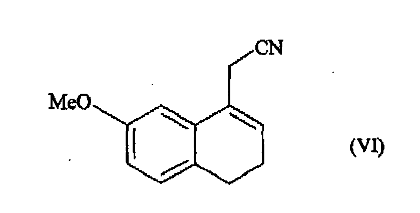 composé de formule (VI) qui est mis en réaction avec un catalyseur d'hydrogénation en présence d'un dérivé allylique pour conduire au composé de formule (VII) : 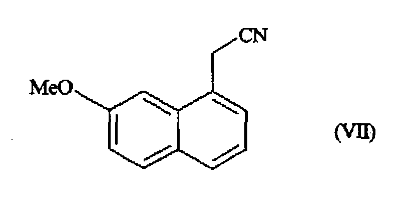 qui est ensuite soumis à une réduction par l'hydrogène en présence de Nickel de Raney dans un milieu éthanol ammoniacal, puis salifié avec de l'acide chlorhydrique pour conduire au composé de formule (VIII) : 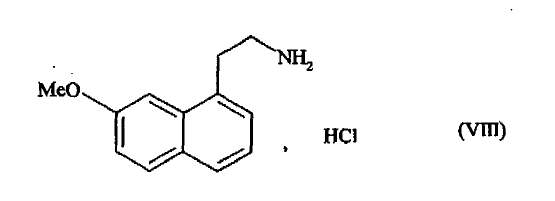 qui est successivement soumis à l'action d'acétate de sodium puis d'anhydride acétique pour conduire au composé de formule (I) que l'on isole sous la forme d'un solide,
étant entendu que :
- par aryle on entend un groupement phényle, naphtyle ou biphényle,
- le terme « substitué » affecté aux expressions « aryle » et « arylalkyle » signifie que la partie aromatique de ces groupements peut être substituée par 1 à 3 groupements, identiques ou différents, choisis parmi alkyle (C₁-C₆) linéaire ou ramifié, hydroxy, et alkoxy (C₁-C₆) linéaire ou ramifié
- par « dérivé allylique » on entend toute molécule contenant 3 à 10 atomes de carbone et pouvant contenir en plus 1 à 5 atomes d'oxygène, et contenant au moins un motif -CH₂-CH=CH₂.

2. Procédé de synthèse du composé de formule (I) selon la revendication 1 **caractérisé en ce que** la réaction de transformation du composé de formule (III) en composé de formule (VI) est réalisée au reflux du toluène.

3. Procédé de synthèse du composé de formule (I) selon la revendication 1, **caractérisé en ce que** R représente un groupement hexyle.

4. Procédé de synthèse du composé de formule (I) selon la revendication 1, **caractérisé en ce que** R' représente un groupement benzyle.

5. Procédé de synthèse du composé de formule (I) selon la revendication 1, **caractérisé en ce que** le catalyseur utilisé pour la réaction de transformation du composé de formule (III) en composé de formule (VI) est le composé de formule (Vₐ) : 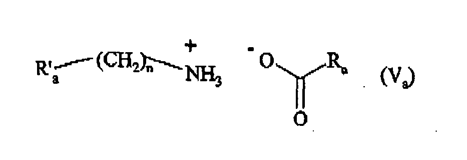 dans laquelle R'ₐ représente un groupement phényle non substitué ou substitué par un ou plusieurs groupements alkyle (C₁-C₆) linéaire ou ramifié, n vaut 0 ou 1, et Rₐ représente un groupement alkyle (C₃-C₁₀) linéaire.

6. Procédé de synthèse du composé de formule (I) selon la revendication 1, **caractérisé en ce que** le catalyseur utilisé pour la réaction de transformation du composé de formule (III) en composé de formule (VI) est l'heptanoate de benzylammonium de formule (IX) :

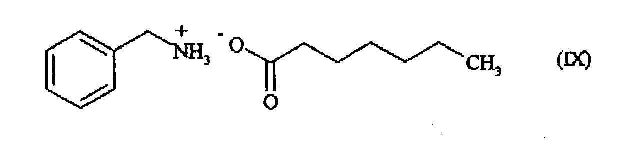

7. Procédé de synthèse du composé de formule (I) selon la revendication 1 **caractérisé en ce que** la réaction de transformation du composé de formule (VI) en composé de formule (VII) est réalisée au reflux du toluène.

8. Procédé de synthèse du composé de formule (I) selon la revendication 1, **caractérisé en ce que** le catalyseur d'hydrogénation utilisé dans la réaction de transformation du composé de formule (VI) en composé de formule (VII) est le palladium.

9. Procédé de synthèse du composé de formule (I) selon la revendication 1, **caractérisé en ce que** le catalyseur d'hydrogénation utilisé dans la réaction de transformation du composé de formule (VI) en composé de formule (VII) est le palladium sur charbon à 5%.

10. Procédé de synthèse du composé de formule (I) selon la revendication 1, **caractérisé en ce que** la quantité de catalyseur d'hydrogénation utilisée dans la réaction de transformation du composé de formule (VI) en composé de formule (VII) est de 5% en poids de catalyseur par rapport au poids de substrat.

11. Procédé de synthèse du composé de formule (I) selon la revendication 1, **caractérisé en ce que** la réaction de transformation du composé de formule (VII) en composé de formule (VIII) est réalisée à 40°C.

12. Procédé de synthèse du composé de formule (I) selon la revendication 1, **caractérisé en ce que** la réaction de transformation du composé de formule (VIII) en composé de formule (I) est réalisée dans l'éthanol.

13. Procédé de synthèse de l'agomélatine à partir du composé de formule (VI), **caractérisé en ce que** le composé de formule (VI) est obtenu par le procédé de synthèse selon l'une quelconque des revendications 1 à 6, qui est mis en réaction avec un catalyseur d'hydrogénation en présence d'un dérivé allylique pour conduire au composé de formule (VII): 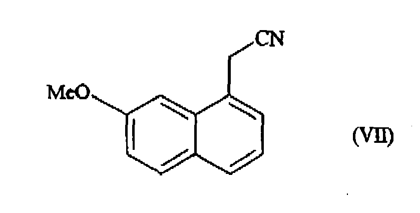 qui est ensuite soumis à une réduction par l'hydrogène en présence de Nickel de Raney dans un milieu éthanol ammoniacal, puis salifié avec de l'acide chlorhydrique pour conduire au composé de formule (VIII) : 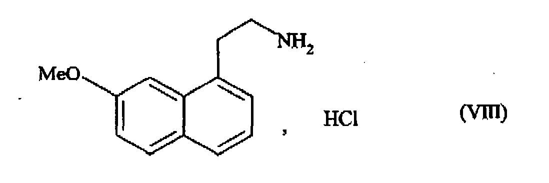 qui est successivement soumis à l'action d'acétate de sodium puis d'anhydride acétique pour conduire au composé de formule (I) que l'on isole sous la forme d'un solide.

14. Procédé de synthèse de l'agomélatine à partir du composé de formule (VII), **caractérisé en ce que** le composé de formule (VII) est obtenu par le procédé de synthèse selon l'une quelconque des revendications 1 à 6 et 7 à 10, qui est ensuite soumis à une réduction par l'hydrogène en présence de Nickel de Raney dans un milieu éthanol ammoniacal, puis salifié avec de l'acide chlorhydrique pour conduire au composé de formule (VIII) : 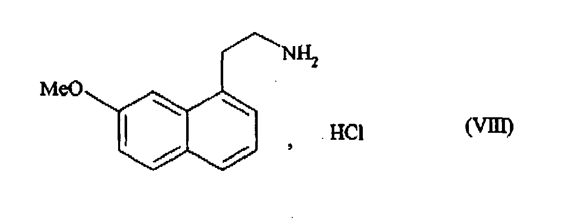 qui est successivement soumis à l'action d'acétate de sodium puis d'anhydride acétique pour conduire au composé de formule (I) que l'on isole sous la forme d'un solide.

15. Procédé de synthèse de l'agomélatine à partir du composé de formule (VIII), **caractérisé en ce que** le composé de formule (VIII) est obtenu par le procédé de synthèse selon l'une quelconque des revendications 1 à 6 et 7 à 11, qui est successivement soumis à l'action d'acétate de sodium puis d'anhydride acétique pour conduire au composé de formule (I) que l'on isole sous la forme d'un solide.

16. Forme cristalline II de l'agomélatine de formule (I) : 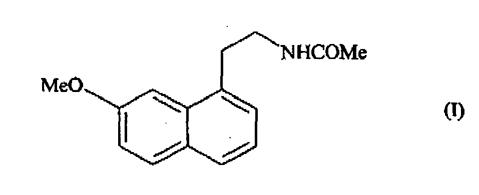 **caractérisée par** les paramètres suivants, obtenus à partir du diagramme de poudre effectué sur le diffractomètre haute résolution D8 de Bruker AXS avec un domaine angulaire 3°-90° en 2θ, un pas de 0,01° et 30 s par pas :
- maille cristalline monoclinique,
- paramètres de maille : a = 20,0903 Å, b = 9,3194 Å, c = 15,4796 Å, β =108,667°
- groupe d'espace : P2₁/n
- nombre de molécules dans la maille : 8
- volume de la maille : Vₘₐᵢₗₗₑ = 2746,742 Å³
- densité : d = 1,13 g/cm³.

17. Compositions pharmaceutiques contenant comme principe actif la forme cristalline II de l'agomélatine selon la revendication 16, en combinaison avec un ou plusieurs véhicules inertes, non toxiques et pharmaceutiquement acceptables.

18. Compositions pharmaceutiques selon la revendication 17 utiles pour la fabrication de médicaments pour traiter les troubles du système mélatoninergique.

19. Compositions pharmaceutiques selon la revendication 17 utiles pour la fabrication de médicaments pour le traitement des troubles du sommeil, du stress, de l'anxiété, des dépressions saisonnières ou de la dépression majeure, des pathologies cardiovasculaires, des pathologies du système digestif, des insomnies et fatigues dues aux décalages horaires, de la schizophrénie, des attaques de paniques, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, des troubles de la circulation cérébrale, ainsi que dans les dysfonctionnements sexuels, en tant qu'inhibiteurs de l'ovulation, d'immunomodulateurs et dans le traitement des cancers.

## Claims

1. Industrial process for the synthesis of the compound of formula (I) 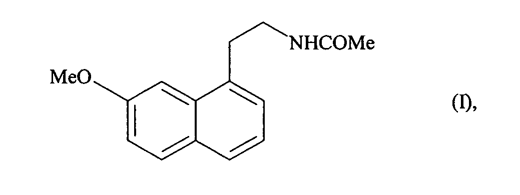 **characterised in that** 7-methoxy-1-tetralone of formula (III) : 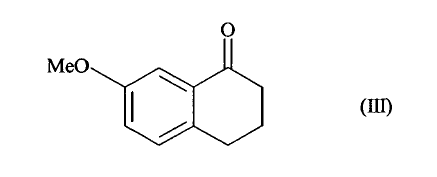 is reacted with cyanoacetic acid of formula (IV) : 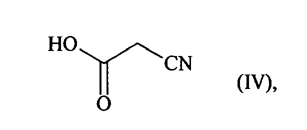 under conditions in which the water formed is removed, in the presence of a catalytic amount of a compound of formula (V) : 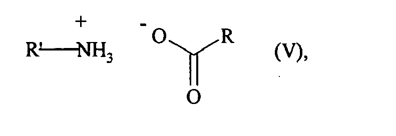 wherein R and R', which may be identical or different, each represent a linear or branched (C₃-C₁₀)alkyl group, an unsubstituted or substituted aryl group or an unsubstituted or substituted aryl-(C₁-C₆)alkyl group in which the alkyl moiety may be linear or branched, to yield, after filtration and washing with a basic solution, (7-methoxy-3,4-dihydro-1-naphthalenyl)acetonitrile of formula (VI) : 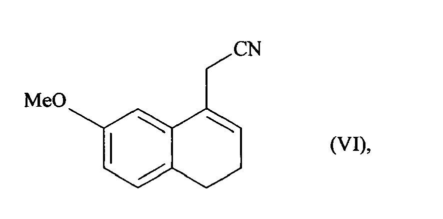 which compound of formula (VI) is reacted with a hydrogenation catalyst in the presence of an allyl compound to yield the compound of formula (VII) : 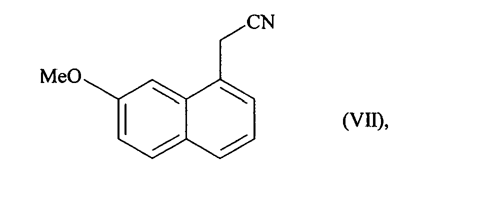 which is then subjected to reduction with hydrogen in the presence of Raney nickel in ammoniacal ethanol medium, and subsequently converted to a salt using hydrochloric acid to yield the compound of formula (VIII) : 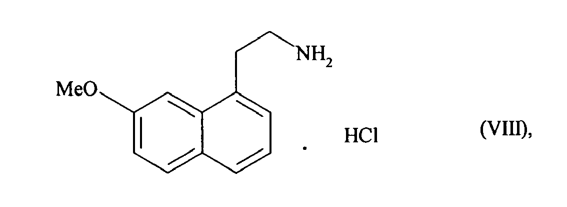 which is subjected successively to the action of sodium acetate and then acetic anhydride to yield the compound of formula (I), which is isolated in the form of a solid,
wherein :
- aryl is understood to be a phenyl, naphthyl or biphenyl group,
- the term "substituted" governing the terms "aryl" and "arylalkyl" denotes that the aromatic moiety of those groups may be substituted by from 1 to 3 identical or different groups selected from linear or branched (C₁-C₆)alkyl, hydroxy and linear or branched (C₁-C₆)alkoxy,
- "allyl compound" is understood to be any molecule containing from 3 to 10 carbon atoms and capable of containing, in addition, from 1 to 5 oxygen atoms, and containing at least one -CH₂-CH=CH₂ moiety.

2. Process for the synthesis of the compound of formula (I) according to claim 1, **characterised in that** the conversion of the compound of formula (III) to the compound of formula (VI) is carried out with reflux of toluene.

3. Process for the synthesis of the compound of formula (I) according to claim 1, **characterised in that** R represents a hexyl group.

4. Process for the synthesis of the compound of formula (I) according to claim 1, **characterised in that** R' represents a benzyl group.

5. Process for the synthesis of the compound of formula (I) according to claim 1, **characterised in that** the catalyst used for the conversion of the compound of formula (III) to the compound of formula (VI) is a compound of formula (Vₐ) : 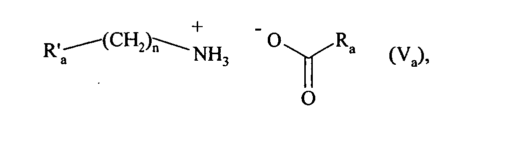 wherein R'ₐ represents a phenyl group unsubstituted or substituted by one or more linear or branched (C₁-C₆)alkyl groups, n is 0 or 1, and Rₐ represents a linear (C₃-C₁₀)alkyl group.

6. Process for the synthesis of the compound of formula (I) according to claim 1, **characterised in that** the catalyst used for the conversion of the compound of formula (III) to the compound of formula (VI) is benzylammonium heptanoate of formula (IX) :

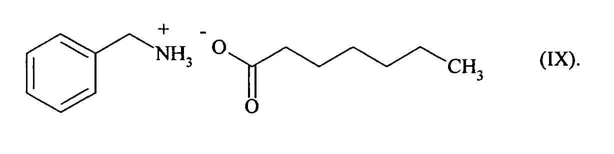

7. Process for the synthesis of the compound of formula (I) according to claim 1, **characterised in that** the conversion of the compound of formula (VI) to the compound of formula (VII) is carried out with reflux of toluene.

8. Process for the synthesis of the compound of formula (I) according to claim 1, **characterised in that** the hydrogen catalyst used in the conversion of the compound of formula (VI) to the compound of formula (VII) is palladium.

9. Process for the synthesis of the compound of formula (I) according to claim 1, **characterised in that** the hydrogenation catalyst used in the conversion of the compound of formula (VI) to the compound of formula (VII) is 5 % palladium-on-carbon.

10. Process for the synthesis of the compound of formula (I) according to claim 1, **characterised in that** the amount of hydrogenation catalyst used in the conversion of the compound of formula (VI) to the compound of formula (VII) is 5% by weight of catalyst in relation to the weight of substrate.

11. Process for the synthesis of the compound of formula (I) according to claim 1, **characterised in that** the conversion of the compound of formula (VII) to the compound of formula (VIII) is carried out at 40°C.

12. Process for the synthesis of the compound of formula (I) according to claim 1, **characterised in that** the conversion of the compound of formula (VIII) to the compound of formula (I) is carried out in ethanol.

13. Process for the synthesis of agomelatine starting from the compound of formula (VI), **characterised in that** the compound of formula (VI) is obtained by the synthesis process according to any one of claims 1 to 6, which compound is reacted with a hydrogenation catalyst in the presence of an allyl compound to yield the compound of formula (VII) : 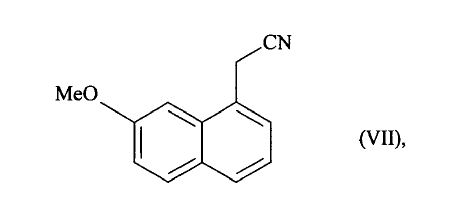 which is then subjected to reduction with hydrogen in the presence of Raney nickel in ammoniacal ethanol medium, and subsequently converted to a salt using hydrochloric acid to yield the compound of formula (VIII) : 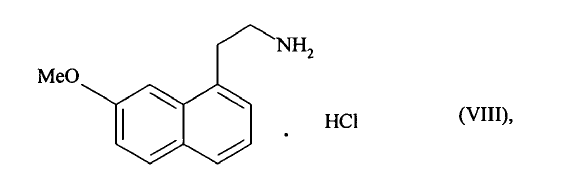 which is subjected successively to the action of sodium acetate and then acetic anhydride to yield the compound of formula (I), which is isolated in the form of a solid.

14. Process for the synthesis of agomelatine starting from the compound of formula (VII), **characterised in that** the compound of formula (VII) is obtained by the synthesis process according to any one of claims 1 to 6 and 7 to 10, and is subsequently subjected to reduction with hydrogen in the presence of Raney nickel in ammoniacal ethanol and then converted to a salt using hydrochloric acid to yield the compound of formula (VIII) : 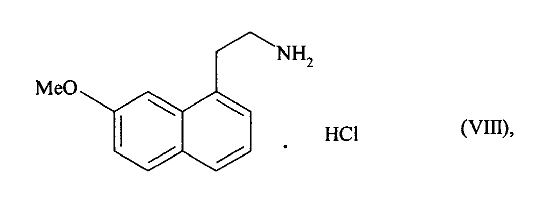 which is subjected successively to the action of sodium acetate and then acetic anhydride to yield the compound of formula (I), which is isolated in the form of a solid.

15. Process for the synthesis of agomelatine starting from the compound of formula (VIII), **characterised in that** the compound of formula (VIII) is obtained by the synthesis process according to any one of claims 1 to 6 and 7 to 11, and is subjected successively to the action of sodium acetate and then acetic anhydride to yield the compound of formula (I), which is isolated in the form of a solid.

16. Crystal form II of agomelatine of formula (I) : 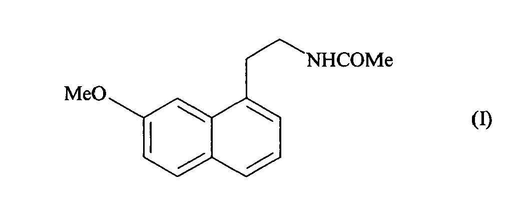 **characterised by** the following parameters, obtained from the powder diagram produced using a Bruker AXS D8 high-resolution diffractometer having an angular field 2θ of 3°-90°, a step of 0.01° and 30 s per step :
- monoclinic crystal lattice,
- lattice parameters : a = 20.0903 Å, b = 9.3194 Å, c = 15.4796 Å, β = 108.667°
- space group : P2₁/n
- number of molecules in the lattice : 8
- lattice volume : V_{lattice} = 2746.742 Å³
- density : d = 1.13 g/cm³.

17. Pharmaceutical compositions comprising, as active ingredient, the crystal form II of agomelatine according to claim 16, in combination with one or more pharmaceutically acceptable, inert, non-toxic carriers.

18. Pharmaceutical compositions according to claim 17 for use in the preparation of medicaments for the treatment of disorders of the melatoninergic system.

19. Pharmaceutical compositions according to claim 17 for use in the preparation of medicaments for the treatment of sleep disorders, stress, anxiety, seasonal affective disorders or major depression, cardiovascular pathologies, pathologies of the digestive system, insomnia and fatigue resulting from jetlag, schizophrenia, panic attacks, melancholia, appetite disorders, obesity, insomnia, psychotic disorders, epilepsy, diabetes, Parkinson's disease, senile dementia, various disorders associated with normal or pathological ageing, migraine, memory losses, Alzheimer's disease, cerebral circulation disorders, and also in the treatment of sexual dysfunctions, as ovulation inhibitors, immunomodulators and in the treatment of cancers.

## Patentansprüche

1. Verfahren zur industriellen Synthese der Verbindung der Formel (I): 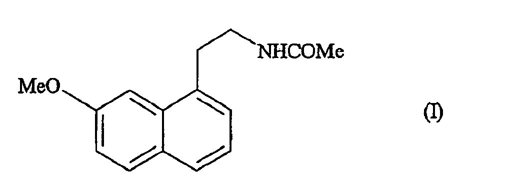 **dadurch gekennzeichnet, dass** man 7-Methoxy-1-tetralon der Formel (III): 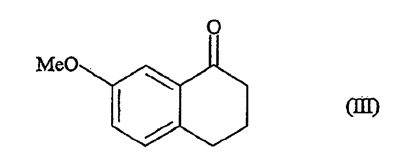 mit Cyanessigsäure der Formel (IV): 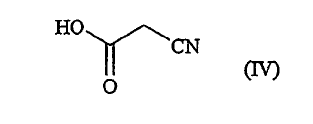 unter Bedingungen der Eliminierung des gebildeten Wassers in Gegenwart einer katalytischen Menge der Verbindung der Formel (V) umsetzt: 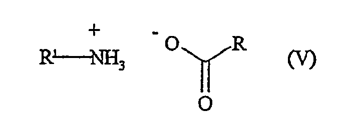 in der R und R', die gleichartig oder verschieden sind, jeweils eine geradkettige oder verzweigte (C₃-C₁₀)-Alkylgruppe, unsubstituierte oder substituierte Arylgruppe oder unsubstituierte oder substituierte, geradkettige oder verzweigte Aryl-(C₁-C₆)-alkylgruppe bedeuten, sodass man nach der Filtration und dem Waschen mit einer basischen Lösung (7-Methoxy-3,4-dihydro-1-naphthalinyl)-acetonitril der Formel (VI) erhält: 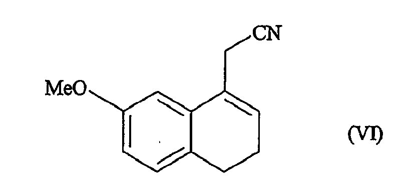 welche Verbindung der Formel (VI) man mit einem Hydrierkatalysator in Gegenwart eines Allylderivats umsetzt zur Bildung der Verbindung der Formel (VII): 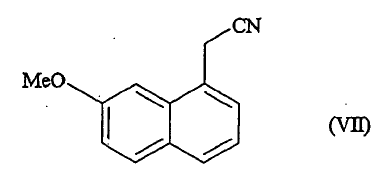 welche anschließend einer Reduktion mit Wasserstoff in Gegenwart von Raney-Nickel in einem ammoniakalischen Ethanolmedium unterworfen und dann mit Chlorwasserstoffsäure in das Salz überführt wird zur Bildung der Verbindung der Formel (VIII): 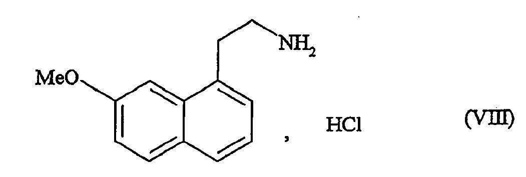 welche nacheinander der Einwirkung von Natriumacetat und dann von Essigsäureanhydrid unterworfen wird zur Bildung der Verbindung der Formel (I), welche man in Form eines Feststoffs isoliert,
mit der Maßgabe, dass man:
- unter Aryl eine Phenyl-, Naphthyl- oder Biphenylgruppe versteht,
- der Begriff "substituiert" in Bezug auf die Bezeichnungen "Aryl" und "Arylalkyl" bedeutet, dass der aromatische Teil dieser Gruppen durch 1 bis 3 gleichartige oder verschiedenartige Gruppen ausgewählt aus geradkettigem oder verzweigtem (C₁-C₆)-Alkyl, Hydroxy und geradkettigem oder verzweigtem (C₁-C₆)-Alkoxy substituiert sein kann und
- unter dem "Allylderivat" jedes Molekül versteht, welches 3 bis 10 Kohlenstoffatome aufweist und zusätzlich 1 bis 5 Sauerstoffatome enthalten kann und mindestens eine Gruppe -CH₂-CH=CH₂ aufweist.

2. Verfahren zur Synthese der Verbindung der Formel (I) nach Anspruch 1, **dadurch gekennzeichnet, dass** die Reaktion der Umwandlung der Verbindung der Formel (III) in die Verbindung der Formel (VI) in Toluol am Rückfluss durchgeführt wird.

3. Verfahren zur Synthese der Verbindung der Formel (I) nach Anspruch 1, **dadurch gekennzeichnet, dass** R eine Hexylgruppe bedeutet.

4. Verfahren zur Synthese der Verbindung der Formel (I) nach Anspruch 1, **dadurch gekennzeichnet, dass** R' eine Benzylgruppe bedeutet.

5. Verfahren zur Synthese der Verbindung der Formel (I) nach Anspruch 1, **dadurch gekennzeichnet, dass** der für die Reaktion der Umwandlung der Verbindung der Formel (III) in die Verbindung der Formel (VI) verwendete Katalysator die Verbindung der Formel (Vₐ) ist: 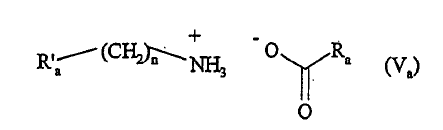 in der R'ₐ eine Phenylgruppe bedeutet, die nicht substituiert ist oder durch eine oder mehrere geradkettige oder verzweigte (C₁-C₆)-Alkylgruppen substituiert ist, n 0 oder 1 bedeutet und Rₐ eine geradkettige (C₃-C₁₀)-Alkylgruppe bedeutet.

6. Verfahren zur Synthese der Verbindung der Formel (I) nach Anspruch 1, **dadurch gekennzeichnet, dass** der für die Reaktion der Umwandlung der Verbindung der Formel (III) in die Verbindung der Formel (VI) verwendete Katalysator Benzylammoniumheptanoat der Formel (IX) ist:

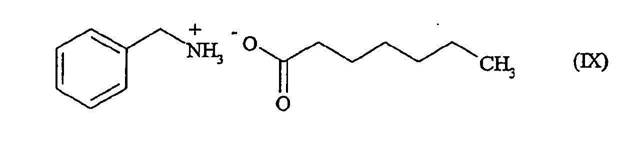

7. Verfahren zur Synthese der Verbindung der Formel (I) nach Anspruch 1, **dadurch gekennzeichnet, dass** die Reaktion der Umwandlung der Verbindung der Formel (VI) in die Verbindung der Formel (VII) in Toluol am Rückfluss erfolgt.

8. Verfahren zur Synthese der Verbindung der Formel (I) nach Anspruch 1, **dadurch gekennzeichnet, dass** der für die Reaktion der Umwandlung der Verbindung der Formel (VI) in die Verbindung der Formel (VII) verwendete Hydrierkatalysator Palladium ist.

9. Verfahren zur Synthese der Verbindung der Formel (I) nach Anspruch 1,
**dadurch gekennzeichnet, dass** der bei der Reaktion der Umwandlung der Verbindung der Formel (VI) in die Verbindung der Formel (VII) verwendete Hydrierkatalysator 5 % Palladium-auf-Kohlenstoff ist.

10. Verfahren zur Synthese der Verbindung der Formel (I) nach Anspruch 1, **dadurch gekennzeichnet, dass** die Menge des bei der Reaktion der Umwandlung der Verbindung der Formel (VI) in die Verbindung der Formel (VII) verwendeten Hydrierkatalysators 5 Gew.-% Katalysator bezogen auf das Gewicht des Substrats, beträgt.

11. Verfahren zur Synthese der Verbindung der Formel (I) nach Anspruch 1, **dadurch gekennzeichnet, dass** die Reaktion der Umwandlung der Verbindung der Formel (VII) in die Verbindung der Formel (VIII) bei 40 °C bewirkt wird.

12. Verfahren zur Synthese der Verbindung der Formel (I) nach Anspruch 1, **dadurch gekennzeichnet, dass** die Reaktion der Umwandlung der Verbindung der Formel (VIII) in die Verbindung der Formel (I) in Ethanol durchgeführt wird.

13. Verfahren zur Synthese von Agomelatin ausgehend von der Verbindung der Formel (VI), **dadurch gekennzeichnet, dass** die Verbindung der Formel (VI) nach dem Syntheseverfahren nach einem der Ansprüche 1 bis 6 hergestellt wird und mit einem Hydrierkatalysator in Gegenwart eines Allylderivats umgesetzt wird zur Bildung der Verbindung der Formel (VII): 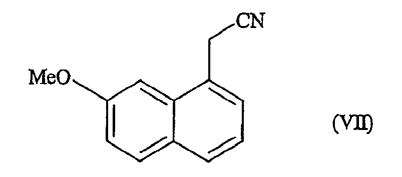 welche anschließend einer Reduktion mit Wasserstoff in Gegenwart von Raney-Nickel in ammoniakalischem Ethanolmedium unterworfen und dann mit Chlorwasserstoffsäure in das Salz überführt wird zur Bildung der Verbindung der Formel (VIII): 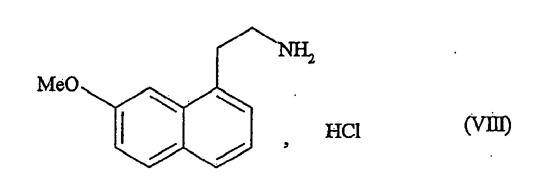 welche nacheinander der Einwirkung von Natriumacetat und dann von Essigsäureanhydrid unterzogen wird zur Bildung der Verbindung der Formel (I), welche man in Form eines Feststoffs isoliert.

14. Verfahren zur Synthese von Agomelatin ausgehend von der Verbindung der Formel (VII), **dadurch gekennzeichnet, dass** die Verbindung der Formel (VII) nach dem Syntheseverfahren nach einem der Ansprüche 1 bis 6 und 7 bis 10 hergestellt wird und anschließend einer Reduktion mit Wasserstoff in Gegenwart von Raney-Nickel in einem ammoniakalischen Ethanolmedium unterworfen wird und dann mit Chlorwasserstoffsäure in das Salz überführt wird zur Bildung der Verbindung der Formel (VIII): 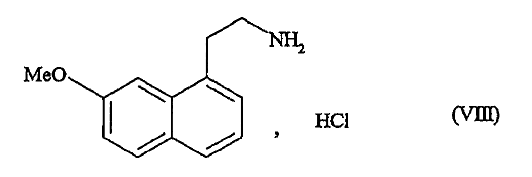 welche nacheinander der Einwirkung von Natriumacetat und dann von Essigsäureanhydrid unterzogen wird zur Bildung der Verbindung der Formel (I), welche man in Form eines Feststoffs isoliert.

15. Verfahren zur Synthese von Agomelatin ausgehend von der Verbindung der Formel (VIII), **dadurch gekennzeichnet, dass** die Verbindung der Formel (VIII) nach dem Syntheseverfahren nach einem der Ansprüche 1 bis 6 und 7 bis 11 hergestellt wird und nacheinander der Einwirkung von Natriumacetat und dann Essigsäureanhydrid unterworfen wird zur Bildung der Verbindung der Formel (I), welche man in Form eines Feststoffs isoliert.

16. Kristallform II von Agomelatin der Formel (I): 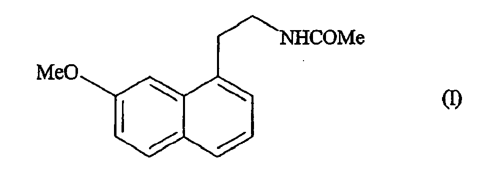 **gekennzeichnet durch** die folgenden Parameter erhalten ausgehend von dem Pulverröntgenbeugungsdiagramm mit Hilfe eines hochauflösenden Diffraktometers D8 von Bruker AXS mit einem Winkelbereich von 3° - 90° 2θ und einer Schrittweite von 0,01° und 30 s pro Schritt:
- monoklines Kristallgitter,
- Parameter der Elementarzelle: a = 20,0903 Å, b = 9,3194 Å, c = 15,4796 Å, β = 108,667°
- Raumgruppe: P2₁/n
- Anzahl von Molekülen in der Elementarzelle: 8
- Volumen der Elementarzelle: Vₘₐᵢₗₗₑ = 2746,742 Å³
- Dichte: d = 1,13 g/cm³.

17. Pharmazeutische Zubereitungen enthaltend als Wirkstoff die Kristallform II von Agomelatin nach Anspruch 16 in Kombination mit einem oder mehreren inerten, nichttoxischen und pharmazeutisch annehmbaren Hilfsstoffen.

18. Pharmazeutische Zubereitungen nach Anspruch 17, nützlich für die Herstellung von Arzneimitteln zur Behandlung von Störungen des melatoninergischen Systems.

19. Pharmazeutische Zubereitungen nach Anspruch 17, nützlich für die Herstellung von Arzneimitteln zur Behandlung von Schlafstörungen, Stress, Angst, saisonal bedingten Depressionen oder starken Depressionen, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, psychischen Störungen, Epilepsie, Diabetes, der Parkinsonschen Krankheit, der senilen Demenz, verschiedenen Störungen, die mit normalen oder pathologischen Altern verbunden sind, der Migräne, Gedächtnisverlusten, der Alzheimerschen Krankheit, Störungen der Gehirndurchblutung sowie von sexuellen Dysfunktionen und als Inhibitoren der Ovulation, Immunomodulatoren und bei der Behandlung von Krebs.
